# Europäisches Patentamt

## European Patent Office

## Office européen des brevets

(11) Veröffentlichungsnummer: **0 212 528**
A2

(12) **EUROPÄISCHE PATENTANMELDUNG**

(21) Anmeldenummer: 86111078.1

(22) Anmeldetag: 11.08.86

(51) Int. Cl.⁴: **G 06 F 15/20**, A 61 B 5/04

(30) Priorität: 30.08.85 CH 3734/85

(71) Anmelder: **Willi Studer AG, Fabrik für elektronische Apparate, Althardstrasse 30, CH-8105 Regensdorf ZH (CH)**

(43) Veröffentlichungstag der Anmeldung: 04.03.87 **Patentblatt 87/10**

(84) Benannte Vertragsstaaten: **AT BE CH DE FR GB IT LI NL SE**

(72) Erfinder: **Schmid, Johann Jakob, Rosengartenstrasse 33, CH-8107 Buchs ZH (CH)**

(54) Verfahren zur Bestimmung von Anfangs- und Endpunkt eines geschlossenen räumlichen Signal-Verlaufes.

(57) Bei einem solchen Verfahren bei dem der Signalverlauf durch Meßsignale dargestellt ist, die mittels Elektroden von einem Lebewesen abgegriffen werden, werden Folgen aufeinanderfolgender räumlicher Steigungswerte des Signalverlaufes gebildet und miteinander korreliert. Als Resultat dieser Korrelierung werden die Folgen Klassen zugeordnet und ein Zeitversatz zwischen den Folgen ermittelt. Für jede Klasse werden die kleinsten Steigungswerte im Anfangs- und Endbereich der Folge ermittelt und in Zeitintervallen um zugehörige Zeitpunkte nach Abtastzeitpunkten gesucht, zu denen Meßwerte gehören, die zueinander im Raum den kleinsten Abstand aufweisen. Die Zeitpunkte dieser Meßwerte ergeben Anfangs- und Endpunkte (20, 21) des Signalverlaufes.

Verfahren zur Bestimmung von Anfangs- und Endpunkt eines geschlossenen raeumlichen Signalverlaufes

--------------------------------------------------------------

Die Erfindung betrifft ein Verfahren zur Bestimmung von Anfangs- und Endpunkt von geschlossenen, raeumlichen Signalverlaeufen, die physiologische Messsignale darstellen, die mittels Elektroden von einem Lebewesen abgegriffen werden.

Ein solches Verfahren ist bereits aus der europaeischen Patentanmeldung 0 150 352 bekannt. Bei diesem Verfahren, das insbesondere zur Bestimmung des Anfangs- und Endpunktes einer QRS-Schleife in einem VKG-Signal vorgesehen ist, werden zunaechst fuer die QRS-Schleife zwei Zeitintervalle definiert. Diesen Zeitintervallen entsprechen Abschnitte der Projektionen der QRS-Schleife in Vektordarstellung. Die beiden Punkte der Abschnitte die den kleinsten raeumlichen Abstand zueinander aufweisen, werden als Nullpunkte der QRS-Schleife bezeichnet. Sie entsprechen den Anfangs- und Endpunkten der QRS-Schleife.

Mit diesem bekannten Verfahren ist es moeglich mit grosser Genauigkeit diese Anfangs- und Endpunkte zu ermitteln. Der rechnerische Aufwand dazu ist aber recht gross, da zwischen einer groesseren Anzahl von Abtastwerten des digitalisierten Signales die raeumlichen Abstaende ermittelt werden muessen. Obwohl diese Berechnungen durch leistungsfaehige Rechner gut durchgefuehrt werden koennen, ist es wuenschbar, den Rechenaufwand zu reduzieren.

Die Erfindung wie sie in den Anspruechen gekennzeichnet ist, loest die Aufgabe ein Verfahren zu schaffen, das es erlaubt mit geringem Rechenaufwand die Anfangs- und Endpunkte geschlossener raeumlicher Signalverlaeufe mit grosser Sicherheit zu bestimmen.

Die durch die Erfindung erreichten Vorteile sind insbesondere darin zu sehen, dass der Rechenaufwand erheblich verkleinert

2

0212528

werden kann. So muss nun der kleinste Abstand zwischen Messwerten einer geschlossenen Signalfolge aus einer wesentlich kleineren Anzahl von Messwerten gebildet werden, weil die Umgebung eines solchen Anfangs- oder Endpunktes bereits genauer vorbekannt ist. Daraus ergibt sich auch eine groessere Sicherheit darin, dass auch wirklich solche Anfangs- und Endpunkte bestimmt wurden. Der bekannte J-Punkt laesst sich dabei ebenfalls leicht ermitteln. Das erfindungsgemaesse Verfahren erlaubt eine Klassierung der einzelnen ermittelten Signalfolgen, was es erleichtert, die Bedeutung der einzelnen Signalfolgen zu ermitteln.

Im folgenden wird die Erfindung anhand von lediglich einen Ausfuehrungsweg darstellenden Zeichnungen naeher erlaeutert. Es zeigt

Figur 1 zeigt ein physiologisches Messsignal in einer bestimmten Ebene mit einer Zeitachse,

Figur 2 zeigt ein physiologisches Messsignal in raeumlicher Darstellung und in Projektionen auf verschiedene Ebenen,

Figur 3 a, b, c und d zeigt ein Flussdiagramm des erfindungsgemaessen Verfahrens,

Figur 4 zeigt ein vom physiologischen Messsignal abgeleitetes Signal,

Figur 5 zeigt ein vereinfachtes Signal gemaess Figur 4, und die

Figuren 6 und 7 zeigen Signale die aus dem Signal gemaess Figur 5 abgeleitet sind.

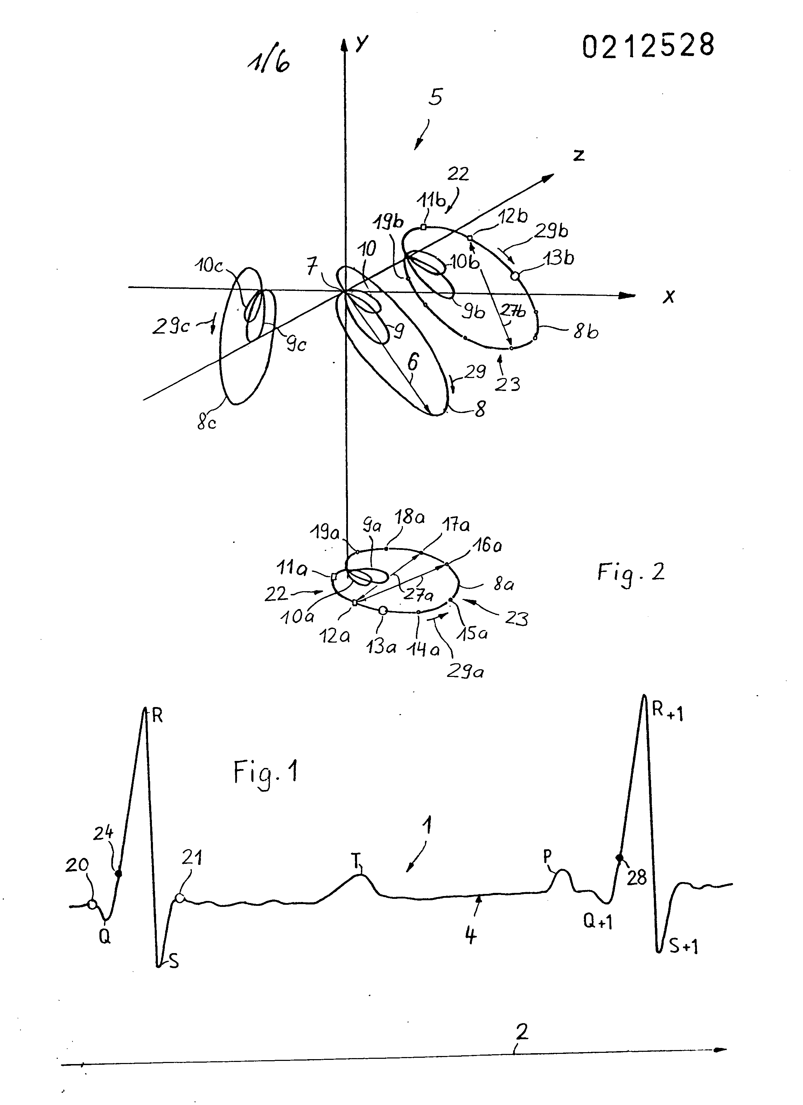

Figur 1 zeigt als physiologisches Messsignal beispielsweise einen Ausschnitt aus einer VKG-Kurve 1. Dabei sind elektrische Potentialwerte, die mit Elektroden von einem Patienten abgegriffen werden ueber einer Zeitachse 2 aufgetragen. Da das erfindungsgemaesse Verfahren am Beispiel eines zeitlich staendig seinen Betrag und seine Richtung aendernden Vektors, der die Taetigkeit des Herzens eines Lebewesens darstellt, erklaert werden soll, koennen diese Potentialwerte als Betrag, des in eine Ebene projizierten Vektors aufgefasst werden.

Ein solcher Vektor bzw. die seinem Endpunkt zugeordneten Potentialwerte beschreiben im zeitlichen Ablauf nacheinander Signalverlaeufe, die beispielsweise als QRS-Welle, T-Welle und P-Welle dem Kardiologen bekannt sind. Zwischen diesen Wellen sind mehr oder weniger kurze und mehr oder weniger konstante Potentialwerte anzeigende Teilstrecken 4 der Kurve 1 eingeschoben.

Figur 2 zeigt in einem dreiachsigen Koordinatensystem 5 den Vektor beispielsweise zu einem bestimmten Zeitpunkt als Pfeil 6 dargestellt. Dieser Vektor geht von einem Nullpunkt 7 aus und endet in Punkten im Raum, die zusammen Schleifen 8, 9 und 10 bilden. Dabei wird die Schleife 8 auch als QRS-Schleife, die Schleife 9 als T-Schleife und die Schleife 10 als P-Schleife bezeichnet. Durch die X-, Y- und Z-Achse des Koordinatensystems 5 werden je eine XY-, XZ- und eine YZ-Ebene gegeben. Die Schleifen 8, 9 und 10 lassen sich in diese drei Ebenen projizieren so, dass weitere Schleifen 8 a, b, c, 9 a, b, c und 10 a, b, c erkennbar sind. Zur Erleichterung nachfolgender Erlaeuterungen sind auch einzelne Punkte 11 a, b bis 19 a, b laengs der Schleifen 8 a und 8 b eingetragen.

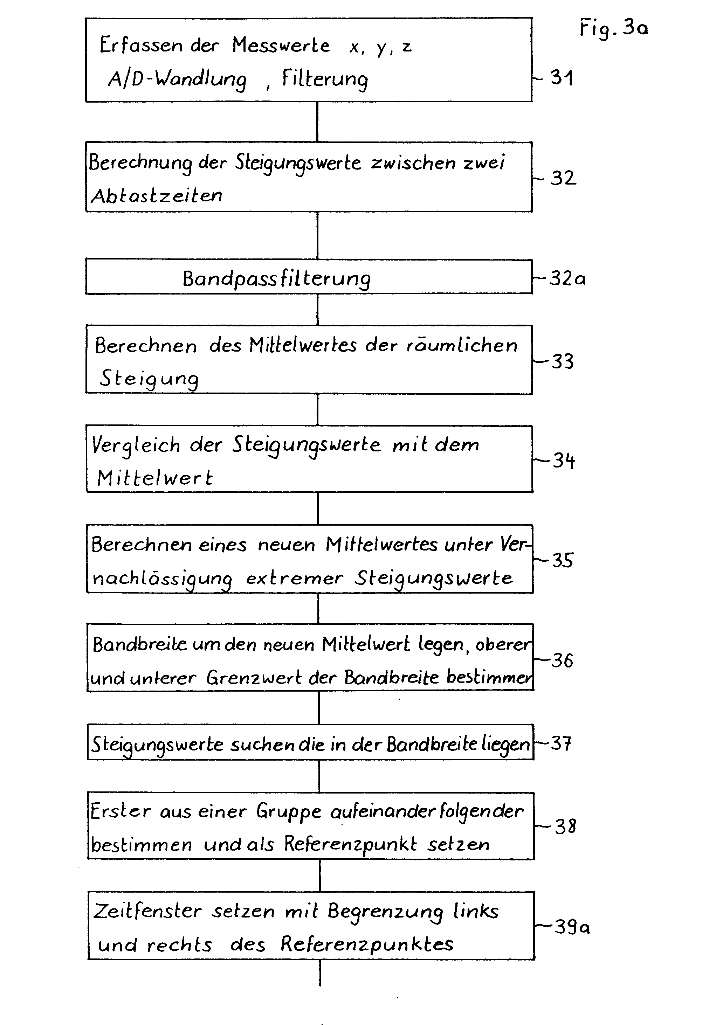

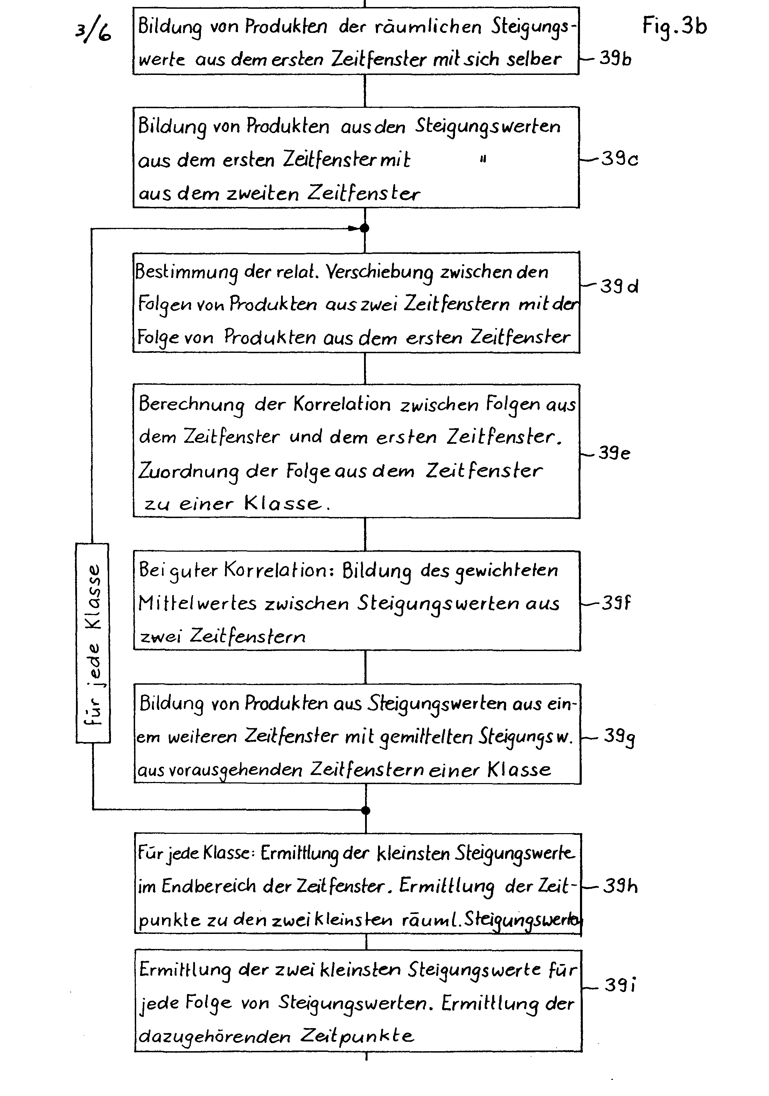

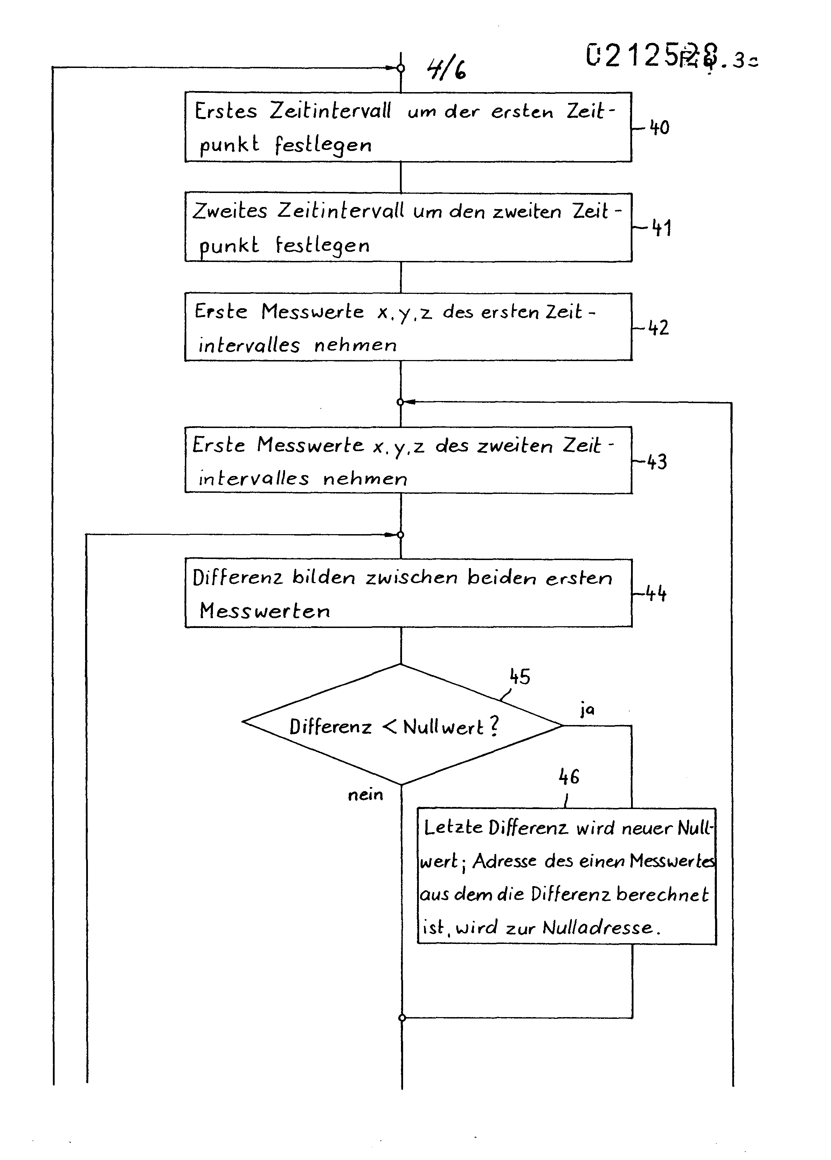

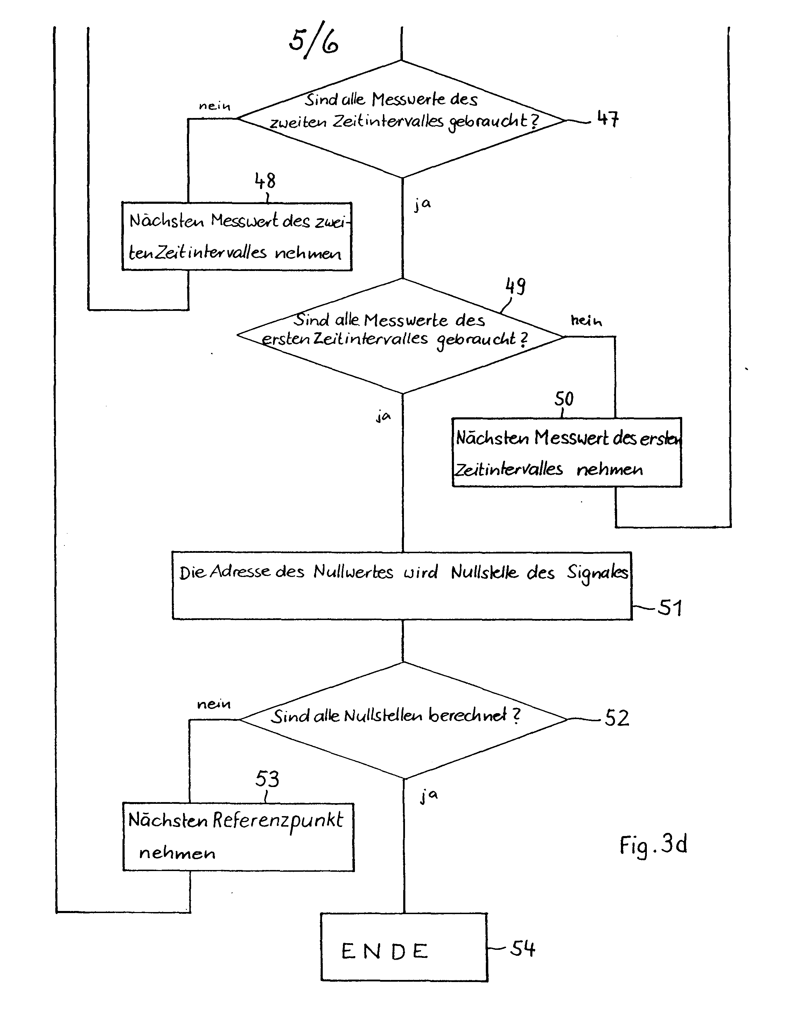

Die Figuren 3a, 3b, 3c und 3d zeigen ein zusammenhaengendes Flussdiagramm mit Kurzbezeichnungen fuer die einzelnen Schritte des erfindungsgemaessen Verfahrens. Diese einzelnen Schritte sind mit Bezugsziffern 31 bis 54 bezeichnet. Dieses Flussdiagramm spricht fuer sich und soll deshalb an dieser Stelle nicht weiter behandelt werden. Es wird aber bei der spaeter folgenden Beschreibung der Wirkungsweise des Verfahrens darauf hingewiesen werden.

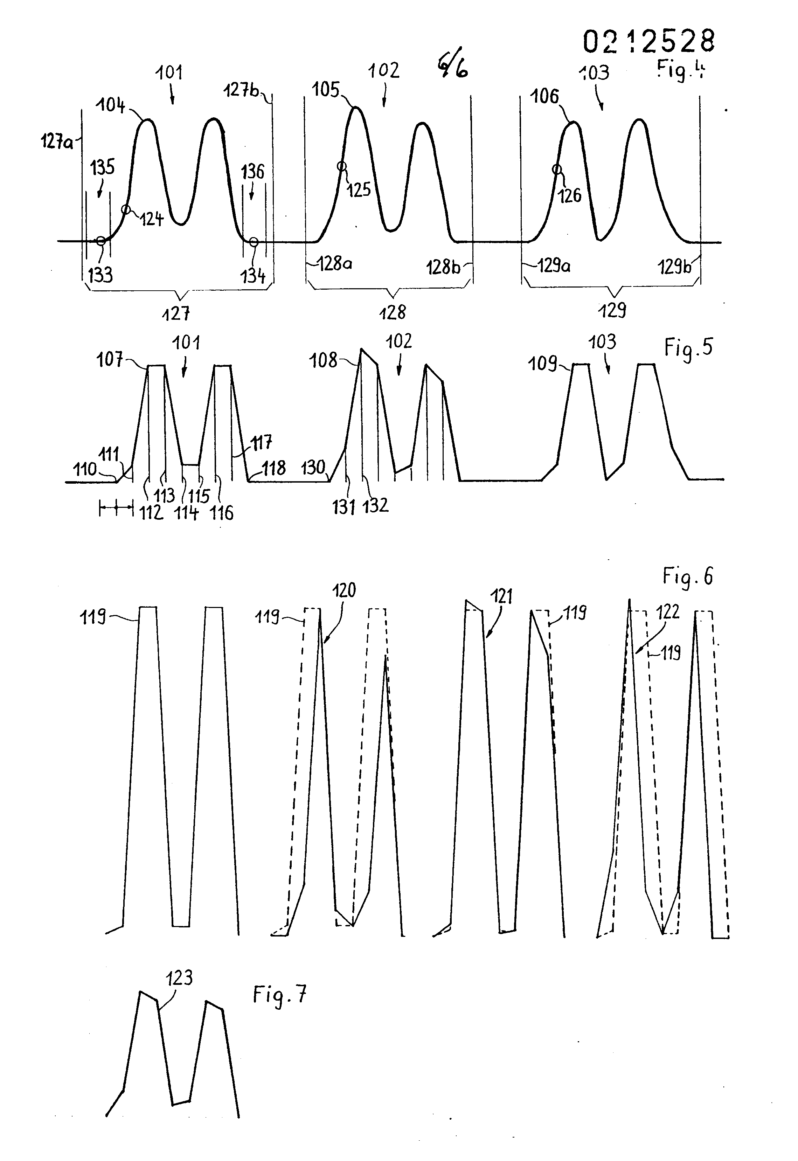

Figur 4 zeigt beispielsweise fuer drei aufeinanderfolgende Herzzyklen 101, 102 und 103 Signale 104, 105 und 106, die die raeumlichen Steigungswerte des Vektors in seiner QRS-Schleife 8 darstellen. Die P- und T-Wellen der Herzzyklen 101, 102 und 103 sind hier vernachlaessigt.

Figur 5 zeigt fuer dieselben Herzzyklen 101, 102 und 103 graphisch vereinfachte Signale 107, 108 und 109, die den Signalen 104, 105 und 106 entsprechen und von diesen der einfacheren spaeteren Erlaeuterung wegen abgeleitet sind. Fuer alle Signale gilt, aber nur fuer die Signale 107 und 108 ist es eingezeichnet, dass die Signale 104 bis 109 aus Folgen von aufeinanderfolgenden, aus digitalen Abtastwerten der QRS-Schleife errechneten, raeumlichen Steigungswerten des Vektors zusammengesetzt sind. Solche digital erfasste raeumliche Steigungswerte sind mit Bezugsziffern 110 bis 118 und 130 bis 132 bezeichnet.

Figur 6 zeigt ein weiteres Signal 119, das aus einer Folge von Produkten aus den raeumlichen Steigungswerten des Signales 107 in Figur 5 besteht. Dazu wird jeder Steigungswert 111 bis 118 mit sich selbst multipliziert und an der selben Stelle aufgetragen. Durch Multiplikation der raeumlichen Steigungswerte des Signales 107 mit den raeumlichen Steigungswerten des Signales 108 entstehen weitere Signale 120, 121 und 122. Dadurch, dass die einzelnen raeumlichen Steigungswerte des Signales 107 um einen Zeitversatz oder ein Abtastintervall 137 nach vorne verschoben werden und anschliessend mit den raeumlichen Steigungswerten des Signales 108 multipliziert werden, entsteht das Signal 120. Ohne zeitliche Verschiebung der raeumlichen Steigungswerte des Signales 107 entsteht das Signal 121. Mit zeitlicher Verschiebung der raeumlichen Abtastwerte im Bereiche des Signales 107 um einen Abtastwert 138 nach hinten, entsteht das Signal 122.

Figur 7 zeigt ein Signal 123 das durch gewichtete Mittelung entsprechender raeumlicher Steigungswerte der Signale 107 und 108 entstanden ist.

Im folgenden werden nun die einzelnen Verfahrensschritte beschrieben, die es erlauben, Anfangs- und Endpunkte beispielsweise einer QRS-Schleife zu bestimmen. Da der Vektor jeweils nacheinander eine QRS-, eine T- und eine P-Schleife beschreibt, ist es wichtig zu wissen, wo und wann beispielsweise die QRS-Schleife beginnt und endet, denn die QRS-Schleife gibt dem Arzt Hinweise auf bestimmte Aspekte der Herztaetigkeit und die T- oder die P-Schleifen geben Hinweise auf andere Aspekte der Herztaetigkeit.

Um zuverlaessige Angaben ueber die Lage von Anfangs- und Endpunkten 20 und 21 (Figur 1) der QRS-Welle oder Schleife zu erhalten, ist es unerlaesslich, die Bewegungen des Vektors und somit die QRS-Schleife 8 in mehreren Ebenen zu erfassen. Deshalb werden bei der Erstellung eines Vektorkardiogramms in an sich bekannter Weise periodisch oder kontinuierlich Werte des Potentiales, das durch den raeumlichen Vektor dargestellt wird, ermittelt. Die graphische Darstellung solcher Werte welche ueber einer Zeitachse 2 in einer Ebene aufgetragen werden, ergibt eine Kurve 1. Die graphische Darstellung solcher Werte in einer XZ-, einer XY- oder ZY-Ebene, also in einem dreiachsigen Koordinatensystem aufgetragen, ergibt QRS-Schleifen 8a, 8b und 8c.

Bei nicht kontinuierlicher, digitaler Erfassung der Werte des Potentiales werden zu bestimmten Abtastzeitpunkten x-Werte, y-Werte und z-Werte des Potentiales ermittelt und gespeichert. Dabei werden die analog anfallenden Werte des Potentiales in an sich bekannter Weise quantisiert, vorausgehend gefiltert und zu digitalen Werten gewandelt. Dies entspricht dem Schritt 31 gemaess Figur 3a. Eine geeignete Kombination solcher Werte von zwei verschiedenen Achsen ergibt Punkte 11a bis 19a der QRS-Schleife 8a oder beispielsweise Punkte 11b bis 19b einer QRS-Schleife 8b. Dabei sind diejenigen Werte die zu demselben Punkt gehoeren im selben Abtastzeitpunkt ermittelt worden.

Gemaess dem Schritt 32 in Figur 3a wird anschliessend aus den Differenzen der Werte zu je zwei aufeinanderfolgenden Abtastzeitpunkten je ein Steigungswert des Vektors berechnet. Dies geschieht nach der Formel

$$f(x,y,z,t) = \sqrt{\left[x(t+T)-x(t)\right]^2 + \left[y(t+T)-y(t)\right]^2 + \left[z(t+T)-z(t)\right]^2}$$

Dies kann getrennt fuer jede der Ebenen des Koordinatensystems 5 durchgefuehrt werden. So wird beispielsweise ein Steigungswert fuer den Bereich zwischen Punkten 12a und 13a in der X-Z-Projektion, 12b und 13b in der X-Y-Projektion ermittelt usw. Dies wird mit allen abgetasteten Werten der QRS-Schleifen 8a, 8b und 8c durchgefuehrt und man erhaelt fuer jeden Bereich zwischen zwei aufeinanderfolgenden Punkten drei Steigungswerte. Diese Steigungswerte werden einem Bandpassfilter zugefuehrt, so dass extreme Steigungswerte ausgeschlossen werden. Dies entspricht einem Schritt 32a. Aus allen so erhaltenen Steigungswerten wird die raeumliche Steigung und aus allen ermittelten Werten fuer die raeumliche Steigung ein Mittelwert errechnet, wie mit dem Schritt 33 vorgesehen.

Ein Vergleich der einzelnen raeumlichen Steigungswerte mit dem Mittelwert, im Schritt 34, erlaubt es beispielsweise raeumliche Steigungswerte die vom Mittelwert um ein bestimmtes Mass abweichen auszuschliessen und nicht mehr weiter zu beruecksichtigen. Aus den uebriggebliebenen raeumlichen Steigungswerten wird ein neuer Mittelwert mit dem Schritt 35 gebildet.

Um den neuen Mittelwert wird eine Bandbreite gelegt, in der raeumliche Steigungswerte des Vektors vernuenftigerweise liegen sollen. Dies ergibt einen Schritt 36. Dies beispielsweise um beim Herzen auftretende Extrasystolen, die sehr hohe Steigungswerte aufweisen, von den Betrachtungen auszuschliessen. Diese Bandbreite ist durch einen oberen Wert und einen unteren Wert begrenzt.

Anschliessend sucht man, im Schritt 37, diejenigen raeumlichen Steigungswerte die in der Bandbreite liegen heraus und waehlt, im Schritt 38, davon den ersten Steigungswert der in der Bandbreite liegt und zu einer Folge von aufeinanderfolgenden und gleichsinnigen Steigungswerten gehoert. Dieser erste raeumliche Steigungswert in der genannten Bandbreite wird als Trigger- oder Referenzpunkt 124, 125, 126 (Fig. 4) bezeichnet und gespeichert. Jeder QRS-Komplex d.h. jede Folge von raeumlichen Steigungswerten einer QRS-Schleife erhaelt somit einen Referenzpunkt 24. Wesentlich ist in der Folge aber nur der Zeitpunkt zu dem der Referenzpunkt auftritt, also der Referenzzeitpunkt.

Bezogen auf jeden dieser Referenzzeitpunkte wird nun ein Zeitfenster 127, 128, 129 (Fig. 4) gesetzt, was den Schritt 39a ergibt. Dieses Zeitfenster wird durch einen Zeitpunkt 127a, 128a, 129a vor und einen Zeitpunkt 127b, 128b, 129b nach dem Referenzzeitpunkt begrenzt. Das Zeitfenster ist gross genug um die raeumlichen Steigungswerte eines QRS-Komplexes aufzunehmen.

Raeumliche Steigungswerte, die Zeitpunkten zugeordnet sind, die in einem ersten Zeitfenster 127 einer Gruppe mehrerer Zeitfenster 127, 128, 129 usw liegen, werden mit sich selber multipliziert, was im Schritt 39b geschieht. Da jeweils ein Zeitfenster eine gleichgrosse Zahl von Abtastzeitpunkten umfasst, geht es bei diesem Schritt darum, dass nur entsprechende Steigungswerte miteinander multipliziert werden, die in der zeitlichen Reihenfolge der Abtastzeitpunkte zu demselben Abtastzeitpunkt, bezogen auf den Zeitpunkt 127a vor dem Referenzzeitpunkt, gehoeren. Aus beispielsweise hundert raeumlichen Steigungswerten entstehen somit wiederum hundert Produkte von Steigungswerten. Mit den Steigungswerten aus Figur 5 durchgefuehrt bedeutet dies, dass der raeumliche Steigungswert 110, der Steigungswert 111, der Steigungswert 112 usw jeder mit sich selbst multipliziert wird.

Gemaess dem naechsten Schritt 39c werden nun entsprechende raeumliche Steigungswerte aus dem ersten Zeitfenster 127 mit Steigungswerten aus dem zweiten Zeitfenster 128 multipliziert. Vereinfacht dargestellt in Figur 5 bedeutet dies, dass der Steigungswert 110 mit dem Steigungswert 130, der Steigungswert 111 mit dem Steigungswert 131, der Steigungswert 112 mit dem Steigungswert 132 usw multipliziert werden. Diese Multiplikationen zwischen Steigungswerten aus dem ersten und aus dem zweiten Zeitfenster 127 und 128 werden aber gleich mehrfach durchgefuehrt. Dies mit dem Unterschied, dass aus dem einen der beiden Zeitfenster 127 oder 128 jeweils eine oder mehrere andere Folgen von Steigungswerten verwendet werden. Zum Beispiel werden vom Signal 107 einmal die Abtastwerte beginnend mit Abtastwert 111 oder beginnend mit Abtastwert 112 oder weiter beginnend mit vor dem Abtastwert 110 auftretenden Abtastwerten verwendet. Das bedeutet, dass die eine der beiden Folgen von raeumlichen Steigungswerten gegenueber der anderen Folge um jeweils einen oder mehrere Abtastwerte verschoben wird fuer die genannten Multiplikationen. Als Resultat dieser Multiplikationen der raeumlichen Steigungswerte aus den Signalen 107 und 108 entstehen Signale 120, 121 und 122 (Fig. 6) bei Verschiebung bis zu zwei Abtastintervallen. Zum Beispiel ist das Signal 120 durch Vorverschieben des Signales 107 um ein Abtastintervall und das Signal 122 durch Nachverschieben des Signales 107 um ein Abtastintervall entstanden.

Bei der Korrelierung der Folgen von Produkten aus dem Schritt 39c mit der Folge von Produkten aus dem Schritt 39b werden die Folgen der Produkte entsprechend den Signalen 120, 121, 122 usw mit dem Signal 119 bzw der dazugehoerenden Folge von Produkten verglichen. Zu jedem Signal 120, 121, 122 usw gehoert ein Wert fuer die Verschiebung die bei dessen Erzeugung angewendet wurde. Durch diesen Vergleich wird ermittelt, welche Folge oder welches Signal 120, 121, 122 am besten mit dem Signal 119 bzw der diesem zugrunde liegenden Folge uebereinstimmt und wie gross allfaellige Abweichungen sind. Dieses Vorgehen ist allgemein unter dem Begriff Korrelation bekannt und bildet die Schritte 39d und 39 e. Als Resultat der Korrelation erhaelt man eine Angabe darueber, welche der Folgen, die durch Multiplikation der Steigungswerte aus den beiden Zeitfenstern, der Folge der Produkte der Steigungswerte aus dem ersten Zeitfenster allein am naechsten kommt sowie um wieviele Abtastintervalle (Zeitversatz) die betreffende Folge der Steigungswerte fuer diese Folge von Produkten verschoben wurde. Dazu gehoert auch, dass zwischen derjenigen Folge von Produkten der raeumlichen Steigungswerte aus dem ersten und zweiten Zeitfenster, die am besten mit der Folge der Produkte der raeumlichen Steigungswerte aus dem ersten Zeitfenster allein uebereinstimmt, ein Korrelationsfaktor bestimmt wird, der angibt wie gut diese Uebereinstimmung ist. Folgen von Produkten die schlechte Uebereinstimmung zeigen werden ausgeschieden oder einer eigenen Klasse zugewiesen, wo sie unter sich beurteilt werden koennen. Es ist auch moeglich Klassen vorausgehend zu definieren und die Folgen laufend entsprechend dem Grad der Uebereinstimmung zuzuordnen.

Fuer zwei Folgen von Produkten aus raeumlichen Steigungswerten aus aufeinanderfolgenden Zeitfenstern, z.B. aus dem ersten und dem zweiten Zeitfenster 127 und 128, wird eine Folge gewichteter gemittelter Steigungswerte 123 (Fig. 7) ermittelt, sofern sie gut miteinander korrelieren. Dies im Schritt 39f.

Die Folge gewichteter gemittelter raeumlicher Steigungswerte wird in nun bekannter Weise mit den entsprechenden raeumlichen Steigungswerten aus dem weiteren Zeitfenster, d.h. aus dem dritten Zeitfenster 129 multipliziert, so dass entsprechend der Zahl der Abtastintervalle um die die eine Folge gegenueber der anderen Folge verschoben wird, eine Anzahl Folgen von Produkten bekannter Art entstehen. Dies entspricht einem Schritt 39g. Dann werden die Schritte 39d, e, f, g wiederholt. Zunaechst fuer die Folgen derselben Klasse und dann fuer die Folgen der anderen Klassen.

Nachdem die Folgen der raeumlichen Steigungswerte aller Zeitfenster in der beschriebenen Weise verarbeitet wurden, liegen nun fuer die Folgen der Produkte der Steigungswerte Klassen vor, die solche Folgen enthalten. Zu jeder Klasse gehoert auch eine Folge gewichteter und gemittelter raeumlicher Steigungswerte. Dazu kommen fuer jede Klasse auch die einzelnen Folgen der raeumlichen Steigungswerte die einem bestimmten Zeitfenster zugordnet sind. Alle diese Werte sind in einem Speicher gespeichert.

Fuer jede Folge der gewichteten und gemittelten Steigungswerte, d.h. fuer jede Klasse werden diejenigen beiden kleinsten raeumlichen Steigungswerte gesucht die im Bereiche der Zeitpunkte 127a und 127b usw. also im Bereiche der Grenzen oder Enden der Zeitfenster liegen. Diese Steigungswerte muessen aber in einer Umgebung mit einer bestimmbaren Anzahl aehnlich kleiner Steigungswerte liegen um beruecksichtigt zu werden. Zu diesen beiden kleinsten Steigungswerten, von denen jeweils einer am Anfang und einer am Ende der Folge liegen muss, werden die Zeitpunkte ermittelt, zu welchen sie auftreten. Dies ergibt den Schritt 39h.

Ausgehend von den Zeitpunkten in denen die kleinsten raeumlichen Steigungswerte fuer jede Folge von gemittelten Steigungswerten oder jede Klasse auftreten und ausgehend von den ermittelten Verschiebungen (Zeitversatz) fuer die einzelnen Folgen von Steigungswerten werden fuer jede einzelne Folge von Steigungswerten oder jedes Zeitfenster Zeitpunkte ermittelt, zu denen die kleinsten Steigungswerte auftreten. Dies ergibt den Schritt 39 i. Falls nicht sehr hohe Anforderungen an die Genauigkeit der Lage der Anfangs- und Endpunkte gestellt werden, ergeben die so ermittelten Zeitpunkte eine gute Naeherung fuer die Anfangs- und Endpunkte.

Fuer jede Folge von raeumlichen Steigungswerten und zu jedem der beiden nun ermittelten kleinsten Steigungswerte 133 und 134 (Fig. 4) der Folge wird ein kleines Zeitintervall 135 und 136 gelegt. Ein solches Zeitintervall betraegt vorzugsweise m Abtastintervalle (z.B. m=10). Dies ergibt die Schritte 40 und 41.

Messwerte die Zeitpunkten zugeordnet sind, die in den genannten beiden Zeitintervallen liegen werden nun verwendet um zwischen jeweils zwei Messwerten, je einem aus jedem Zeitintervall 135 und 136, die Differenz bzw den raeumlichen Abstand zu bilden. Dies ergibt die Schritte 42, 43 und 44. Bezogen auf Figur 1 bedeutet dies, dass Abstaende 27a, 27b usw zwischen Punkten 11a, 12a, 11b, 12b usw der QRS-Schleife 8a, 8b usw ermittelt werden. Die genannten Punkte und Abstaende sind hier der besseren Uebersichtlichkeit wegen weit weg von den genannten Orten oder Zeiten mit der kleinsten raeumlichen Steigung gezeichnet. Die raeumlichen Abstaende zwischen Endpunkten des Vektors in einem dreiachsigen Koordinatensystem berechnen sich gemaess der bekannten Formel:

$$a = \sqrt{(X1 - X2)^2 + (Y1 - Y2)^2 + (Z1 - Z2)^2}$$

fuer zwei Punkte welche durch Indizes 1 und 2 bezeichnet sind.

Die erhaltenen Werte der raeumlichen Abstaende zwischen den Messwerten, die den Zeiten in den beiden Zeitintervallen 135, 136 zugeordnet sind, werden nach dem kleinsten dieser Abstaende abgesucht. Jedem dieser Messwerte ist ein Abtastzeitpunkt zugeordnet. Diese Abtastzeitpunkte der beiden Punkte, die den kleinsten Abstand zueinander aufweisen, werden nun als Anfangspunkt 20 und Endpunkt 21 der QRS-Welle bzw. als Nullpunkte 7 der QRS-Schleife bezeichnet. Aus dem Flussdiagramm in Figur 3c und 3d sind die einzelnen Schritte 45 bis 54 dazu ersichtlich.

Das eben beschriebene Verfahren eignet sich besonders gut in den Faellen, in denen zuerst alle Daten erfasst und anschliessend ausgewertet werden. Das Verfahren kann aber auch fuer unmittelbare laufende Datenauswertung, die schon waehrend der Datenerfassung erfolgt, angepasst werden. Dazu kann beispielsweise fuer ein erstes erfasstes Signal oder fuer einen ersten Herzzyklus 101 ein fester Steigungswert vorgegeben werden. Der erste ermittelte Steigungswert, der diesen vorgegebenen Steigungswert erreicht, liefert einen Abtastzeitpunkt, der als Referenzzeitpunkt 24 angenommen wird. Ausgehend von diesem Referenzzeitpunkt werden die Anfangs- und Endpunkte der QRS-Welle auf bereits bekannte Art ermittelt.

Fuer das zweite Signal oder den zweiten Herzzyklus 102 der Messreihe kann eine verbesserte Annahme fuer den Referenzzeitpunkt gemacht werden, indem man nach dem Referenzzeitpunkt des ersten Herzzyklus 101 die maximal auftretende gleichsinnige Steigung ermittelt und den dazugehoerigen Abtastzeitpunkt als neuen Referenzzeitpunkt fuer den zweiten Herzzyklus betrachtet.

Es kann weiter die Zeitdauer zwischen den nun bekannten Referenzzeitpunkten 24 und 28 des ersten und des zweiten Herzzyklus gemessen werden und vom Anfangs- und Endpunkt des zweiten Herzzyklus abgetragen werden. So erhaelt man Anfangs- und Endpunkt des zweiten Herzzyklus.

So kann die Ermittlung des Referenzzeitpunktes und daraus folgend die Ermittlung der Anfangs- und Endpunkte der QRS-Welle laufend verbessert werden. Als Resultat dieses Verfahrens steht fuer jeden Herzzyklus oder allgemein fuer jede Periode des betrachteten physiologischen Signales der Anfangs- und der Endpunkt des Signales oder verschiedener Teilsignale in Bezug auf deren zeitliches Auftreten sowie daraus abgeleitet in Bezug auf deren elektrisches Potential einwandfrei fest. Es ist naheliegend, dass dieses Verfahren sich insbesondere zur Auswertung der ermittelten Daten in einer Datenverarbeitungsanlage oder in einem Rechner eignet.

Es besteht mit diesem Verfahren auch die Moeglichkeit den sogenannten J-Punkt zu bestimmen. In Figur 1 entspricht der Punkt 21 auch dem J-Punkt. Dazu wird im hinteren Zeitintervall 134 eines Zeitfensters 127 nach demjenigen ersten raeumlichen Steigungswert gesucht, der eine gesetzte Limite unterschreitet. Zu der Zeit, zu der dieser Steigungswert auftritt, gehoert auch der Messwert des J-Punktes. Bei gesunden Menschen faellt der J-Punkt mit dem Endpunkt zusammen.

0212528

00 Patentansprueche:

1. Verfahren zur Bestimmung von Anfangs- und Endpunkt von geschlossenen, raeumlichen Signalverlaeufen (8, 9, 10), die physiologische Messsignale darstellen, die mittels Elektroden von einem Lebewesen abgegriffen werden, dadurch gekennzeichnet, dass

raeumliche Steigungswerte der Signalverlaeufe ermittelt werden, dass

daraus Folgen (104, 105, 106) von aufeinanderfolgenden raeumlichen Steigungswerten ausgeschieden werden, dass

die raeumlichen Steigungswerte aus diesen Folgen miteinander korreliert werden, dass

diese Folgen als Resultat der Korrelierung Klassen zugeordnet werden, dass

aus den genannten Folgen fuer jede Klasse eine Folge (123) mit gemittelten raeumlichen Steigungswerten gebildet wird, dass

als Resultat der Korrelierung fuer jede Folge ein Zeitversatz (137, 138) zu der Folge mit gemittelten raeumlichen Steigungswerten ermittelt wird, dass

je ein kleinster raeumlicher Steigungswert in beiden Endbereichen der genannten Folge von gemittelten raeumlichen Steigungswerten ermittelt wird, dass

aus den kleinsten raeumlichen Steigungswerten aus der Folge der gemittelten raeumlichen Steigungswerte und aus dem Zeitversatz je ein Zeitpunkt (133, 134) fuer die kleinsten raeumlichen Steigungswerte jeder einzelnen Folge ermittelt wird, die als Anfangs- und Endpunkt gelten.

2. Verfahren gemaess Anspruch 1, dadurch gekennzeichnet, dass um die genannten Zeitpunkte (133, 134) der Signalverlaeufe Zeitbereiche (135, 136) bestimmt werden, dass

zwischen Punkten eines raeumlichen Signalverlaufes die in den genannten Zeitbereichen liegen der raeumliche Abstand ermittelt wird, und dass

die Punkte die den kuerzesten Abstand voneinander aufweisen als Anfangs- und Endpunkt des raeumlichen Signalverlaufes betrachtet werden.

3. Verfahren gemaess Anspruch 1, dadurch gekennzeichnet, dass in der Folge der gemittelten raeumlichen Steigungswerte ein kleinster raeumlicher Steigungswert aus einer Mehrzahl aufeinanderfolgender aehnlich kleiner raeumlicher Steigungswerte der Folge ermittelt wird.

4. Verfahren gemaess Anspruch 1, dadurch gekennzeichnet, dass fuer jede Folge aufeinanderfolgender raeumlicher Steigungswerte je zwei kleinste raeumliche Steigungswerte in den Endbereichen der Folge ermittelt werden.

5. Verfahren gemaess Anspruch 1, dadurch gekennzeichnet, dass jedem, einem kleinsten raeumlichen Steigungswert einer Folge zugeordneten, Zeitpunkt (133, 134) ein Zeitbereich zugeordnet wird, der m Abtastintervalle umfasst.

6. Verfahren gemaess Anspruch 1, dadurch gekennzeichnet, dass Folgen von raeumlichen Steigungswerten von QRS-Komplexen ausgeschieden werden.

7. Verfahren gemaess Anspruch 1, dadurch gekennzeichnet, dass die raeumlichen Steigungswerte gemaess einer Bandpasscharakteristik gefiltert werden.

Fig. 2

Fig. 1

0212528

Fig. 3a

Erfassen der Messwerte x, y, z
A/D-Wandlung , Filterung | — 31

Berechnung der Steigungswerte zwischen zwei
Abtastzeiten | — 32

Bandpassfilterung | — 32a

Berechnen des Mittelwertes der räumlichen
Steigung | — 33

Vergleich der Steigungswerte mit dem
Mittelwert | — 34

Berechnen eines neuen Mittelwertes unter Vernachlässigung extremer Steigungswerte | — 35

Bandbreite um den neuen Mittelwert legen, oberer
und unterer Grenzwert der Bandbreite bestimmen | — 36

Steigungswerte suchen die in der Bandbreite liegen | — 37

Erster aus einer Gruppe aufeinander folgender
bestimmen und als Referenzpunkt setzen | — 38

Zeitfenster setzen mit Begrenzung links
und rechts des Referenzpunktes | — 39a

0212528

Fig. 3b

| Bildung von Produkten der räumlichen Steigungs-werte aus dem ersten Zeitfenster mit sich selber | — 39b |

| Bildung von Produkten aus den Steigungswerten aus dem ersten Zeitfenster mit " aus dem zweiten Zeitfenster | — 39c |

| Bestimmung der relat. Verschiebung zwischen den Folgen von Produkten aus zwei Zeitfenstern mit der Folge von Produkten aus dem ersten Zeitfenster | — 39d |

| Berechnung der Korrelation zwischen Folgen aus dem Zeitfenster und dem ersten Zeitfenster. Zuordnung der Folge aus dem Zeitfenster zu einer Klasse. | — 39e |

| Bei guter Korrelation: Bildung des gewichteten Mittelwertes zwischen Steigungswerten aus zwei Zeitfenstern | — 39f |

| Bildung von Produkten aus Steigungswerten aus ein-em weiteren Zeitfenster mit gemittelten Steigungsw. aus vorausgehenden Zeitfenstern einer Klasse | — 39g |

Für jede Klasse

| Für jede Klasse: Ermittlung der kleinsten Steigungswerte im Endbereich der Zeitfenster. Ermittlung der Zeit-punkte zu den zwei kleinsten räuml. Steigungswerten | — 39h |

| Ermittlung der zwei kleinsten Steigungswerte für jede Folge von Steigungswerten. Ermittlung der dazugehörenden Zeitpunkte | — 39i |

0212528 Fig.3c

```
┌─────────────────────────────────────────┐
│ Erstes Zeitintervall um der ersten Zeit- │ ─40
│ punkt festlegen                          │
└─────────────────────────────────────────┘
                    │
┌─────────────────────────────────────────┐
│ Zweites Zeitintervall um den zweiten Zeit-│ ─41
│ punkt festlegen                          │
└─────────────────────────────────────────┘
                    │
┌─────────────────────────────────────────┐
│ Erste Messwerte x,y,z des ersten Zeit-   │ ─42
│ intervalles nehmen                       │
└─────────────────────────────────────────┘
                    │
┌─────────────────────────────────────────┐
│ Erste Messwerte x,y,z des zweiten Zeit-  │ ─43
│ intervalles nehmen                       │
└─────────────────────────────────────────┘
                    │
┌─────────────────────────────────────────┐
│ Differenz bilden zwischen beiden ersten  │ ─44
│ Messwerten                               │
└─────────────────────────────────────────┘
                    │
              ╱─────────────╲        45
             ╱  Differenz <   ╲  ja
             ╲  Nullwert ?    ╱────────
              ╲─────────────╱
                    │ nein         46
                                   │
        ┌──────────────────────────────────┐
        │ Letzte Differenz wird neuer Null- │
        │ wert; Adresse des einen Messwertes│
        │ aus dem die Differenz berechnet   │
        │ ist, wird zur Nulladresse.        │
        └──────────────────────────────────┘
```

0212528

Sind alle Messwerte des
zweiten Zeitintervalles gebraucht? —47

nein

48

Nächsten Messwert des zweiten Zeitintervalles nehmen

ja

49

Sind alle Messwerte des
ersten Zeitintervalles gebraucht?

nein

ja

50

Nächsten Messwert des ersten
Zeitintervalles nehmen

Die Adresse des Nullwertes wird Nullstelle des Signales

—51

Sind alle Nullstellen berechnet? —52

nein

ja

53

Nächsten Referenzpunkt
nehmen

Fig. 3d

E N D E —54

0212528

6/6

Fig.4

Fig.5

Fig.6

Fig.7